# EUROPEAN PATENT APPLICATION

(11) **EP 4 464 312 A1**
(43) Date of publication of application: **20.11.2024**
(21) Application number: 23752432.7
(22) Date of filing: 10.02.2023
(51) Int. Cl.: A61K 9/06, A23L 33/12, A61P 7/02, A61P 25/28, A61P 19/02, A61P 19/06, A61P 11/06, A61P 29/00, A61P 9/10, A61P 27/10

(54) **OLEOGEL AND PREPARATION METHOD THEREFOR AND USE THEREOF**

(30) Priority: 11.02.2022 CN 202210126905
(71) Applicant: Sirio Pharma Co., Ltd., Shantou, Guangdong 515000 (CN)
(72) Inventor: FANG, Suqiong, Shantou, Guangdong 515000 (CN); CHEN, Wenrong, Shantou, Guangdong 515000 (CN); WANG, Peijian, Shantou, Guangdong 515000 (CN)
(74) Representative: Cabinet Becker et Associés
(86) International application number: PCT/CN2023/075433
(87) International publication number: WO 2023/151653

(57) **Abstract**

An oleogel and a preparation method therefor and a use thereof. The oleogel comprises 60-90 wt% of liquid oil, 6-35 wt% of a structuring agent, and 4-15 wt% of water. The liquid oil comprises an unsaturated fatty acid, and the structuring agent is a protein denatured particle. The oleogel is an antioxidant edible oil composition rich in unsaturated fatty acids, an additional stabilizer does not need to be added, the use of a thickener, an emulsifier, and an organic reagent is avoided, and the oleogel has oxidation resistance and is beneficial to long-term storage of products. The oleogel can be used as an oral pharmaceutical preparation to achieve various therapeutic utility.

## Description

### Technical Field

The present disclosure belongs to the field of functional foods, and in particular relates to a functional composition rich in unsaturated fatty acids, which is used as an oral pharmaceutical preparation to improve the physical condition of a subject, and prevent or treat related diseases or conditions.

### Background of the invention

Unsaturated fatty acid is a fatty acid that constitutes fats in a body and is an indispensable fatty acid for the human body. Unsaturated fatty acids are divided into two types: monounsaturated fatty acids and polyunsaturated fatty acids based on the number of double bonds. As a common animal source of unsaturated fatty acids, fish oil contains unsaturated fatty acids that combine with cholesterol to form esters, which then are degraded into cholic acid and are excreted from the body. It has the ability to significantly reduce blood cholesterol and triglycerides and increase high-density lipoprotein which can be used for anti-hyperlipidemia. In addition, fish oil also has functions such as anti-platelet aggregation, anti-coagulation, and ability to prevent the occurrence of thrombotic diseases.

However, the oxidation of unsaturated fatty acids has always been an important direction that has attracted much attention, especially in the oil industry, pharmaceutical and food industries, etc., which are heavily involved in the oxidation of unsaturated fatty acids. Addressing the issue of unsaturated fatty acid oxidation in the prior art often involves the use of antioxidants. However, for production costs and health purposes, many methods have been tried in the art to avoid the use of antioxidants. Among these methods, the use of a structuring agent to deal with unsaturated fatty acids to form oleogel becomes one of the options.

According to different molecular weights, structuring agents which can be used for oil can be divided into small molecule gelling agents and biological macromolecule gelling agents. Small molecule gelling agents (glyceryl mono- and di-stearate, waxes, lecithin, fatty acids and their derivatives, etc.) usually self-assemble through heating-cooling to form a crystal network structure, thereby structuring the liquid oil to prepare an oil composition. However, this type of process requires the heating temperature above the melting point of the small molecule gelling agent, and higher operating temperatures can easily cause oxidative rancidity of unsaturated fatty acids. Biological macromolecules, especially proteins, have become the best choice as lipid structuring agent due to their high nutritional value and high consumer acceptance. Since hydrophilic proteins are not soluble in oil, this limits the direct application of proteins in the field of oil structuring. Oil structuring using protein as a structuring agent is usually prepared in an indirect way, which mainly includes Pickering emulsion template method, foam template method and solvent replacement method.

Chinese invention patent application (issued as CN107950684) discloses a method for preparing an oil composition rich in unsaturated fatty acids using Pickering emulsion as a template. This method first prepares a gelatin-polyphenol complex solution, and then shears and homogenizes it with vegetable oil rich in unsaturated fatty acids to prepare a mixed emulsion which is then dried and sheared to obtain a solid oil composition. This method requires complex processes such as homogenization, drying, and shearing, and the preparation process is cumbersome. The oil composition finally obtained by this patent contains gelatin, which is not accepted by consumers for various reasons, and includes antioxidant polyphenols and polysaccharides, forming protein-polyphenol-polysaccharides adsorbed on the surface of oil in the form of particles. Furthermore, this method produces oleogel through shearing, the viscoelasticity is difficult to be controlled, and the oil content is not high (less than 60%).

Chinese invention patent application (publication number CN112063002A) discloses a porous material prepared from rice bran protein through a foam template method for adsorbing organic solvents to prepare organic gels. However, the gel uses organic solvents and cannot meet the requirements for the preparation of oleogel. And the product contains more air, which is not conducive to the protection of oil.

Chinese invention patent application (publication number CN105228461A) discloses a method for continuously preparing oil compositions with ethyl cellulose. This method utilizes the property of ethyl cellulose to melt at high temperatures, mixes it with oil in an extruder, and the mixture is cooled to form an oil composition. However, in this method, the temperature of the melting zone of the extruder needs to reach as high as 100~200°C, and the high-temperature process will accelerate the oxidation of unsaturated fatty acids.

There is still a need in the art to optimize a structuring agent to improve the shortcomings of existing oleogels.

### Summary of the invention

In view of the problems existing in the oleogels, that is, low oil content, poor and unmanageable rheological properties and oxidation of unsaturated fatty acids due to factors such as heating during the treatment process, the inventor provides a new oleogel. The preparation process of the oleogel is simple and does not include a heating step that could cause the oxidation of unsaturated fatty acids. The oleogel of the present disclosure also has the following characteristics: high oil content (greater than 60%), suitable rheological properties (having a storage modulus value of 1×10³Pa-1×10⁵Pa and a loss factor of 0.08-0.5) and high antioxidant properties (peroxide value below 2.5mmol/kg).

In one aspect, the present disclosure provides an oleogel comprising 60-90wt% of a liquid oil, 6-35wt% of a structuring agent and 4-15wt% of water, wherein the liquid oil comprises unsaturated fatty acids, the structuring agent is a protein denatured particle, and the liquid oil is a continuous phase.

In one embodiment, the content of the liquid oil is 63-85wt%; and/or the content of the structuring agent is 6-25wt%.

In one embodiment, the unsaturated fatty acid is one or more of a monounsaturated fatty acid and a polyunsaturated fatty acid. The monounsaturated fatty acid may include one or more of myristic acid, palmitoleic acid, oleic acid, trans-oleic acid, ricinoleic acid, erucic acid and cetoleic acid. The polyunsaturated fatty acid may include one or more of omega-3 and omega-6 polyunsaturated fatty acids.

In one embodiment, the protein is one or more of animal proteins and plant proteins. The animal protein may include one or more of whey protein, casein, collagen and gelatin.

In one embodiment, the protein denatured particles are obtained by preparing a protein suspension, heating to denature, homogenizing and drying. Homogenization may be performed by one or more of mechanical stirring homogenization, ultrasonic homogenization, microfluidization homogenization, homogenization with colloid milling, and homogenization with ball milling. Drying may be performed by one or more of vacuum drying, vacuum freeze drying, electric heating blast drying and spray drying.

In one embodiment, the protein suspension is 2-25wt% of whey protein aqueous suspension.

In one embodiment, the liquid oil is one or more of olive oil, canola oil, peanut oil, algae oil, fish oil, linseed oil, perilla seed oil, chia seed oil and krill oil.

In one embodiment, the oleogel contains or does not contain an antioxidant. The antioxidant may be one or more of an oil-soluble antioxidant and a water-soluble antioxidant.

In one embodiment, the oil-soluble antioxidant is one or more of rosmarinol, carnosol, sesamol, vitamin E, butylated hydroxyanisole, dibutyl hydroxytoluene, propyl gallate and tert-butylhydroquinone, and the water-soluble antioxidant is one or more of vitamin C, sodium isoascorbate, tea polyphenols, epigallocatechin gallate, sugars and sugar alcohols; wherein the sugars include sucrose; the sugar alcohols are one or more of xylitol, sorbitol, erythritol and maltitol.

In one embodiment, the oleogel has one or more of the following characteristics as measured by a rheometer: (1) having a storage modulus value of 1×10³Pa-1×10⁵Pa; and (2) having a loss factor of 0.08-0.5.

In another aspect, the present disclosure provides use of the oleogel of the present disclosure in food, health products or cosmetics, or use of the oleogel of the present disclosure in the preparation of an oral pharmaceutical preparation, wherein the oral pharmaceutical preparation is used for one or more of the following: (1) regulating blood lipids and/or clearing thrombi; (2) preventing senile dementia, nourishing the brain and/or improving memory; (3) preventing arthritis, relieving gout and asthma, and temporarily relieving swelling and pain caused by arthritis; (4) improving vision and/or preventing and treating presbyopia; and (5) maintaining the retina.

In another aspect, the present disclosure provides an oral pharmaceutical preparation, which comprises the oleogel of the present disclosure.

In another aspect, a method for preparing the oleogel of the present application is provided, which comprises: (1) adding a structuring agent to a liquid oil and mixing them to obtain an oil phase suspension of the structuring agent; and
(2) adding water to the oil phase suspension of the structuring agent and homogenizing them to obtain an oleogel.

In one embodiment, the method further comprises the step of adding an oil-soluble antioxidant, a water-soluble antioxidant or both to the oil phase, the water phase or both, respectively.

In one embodiment, the method does not include the step of heating the liquid oil.

The advantages of the present disclosure include:
1. The oleogel of the present disclosure has an oil content higher than 60%, which meets the needs of high-oil-content products in the prior art. The oleogel of the present disclosure has solid/semi-solid properties and good rheological properties such as plasticity, spreadability and shear rheology, and can be widely used in food systems.
2. The oleogel of the present disclosure has suitable rheological properties (storage modulus value of 1×10³Pa-1×10⁵Pa and loss factor of 0.08-0.5).
3. The oleogel of the present disclosure has high antioxidant properties (peroxide value below 2.5mmol/kg) and can be stored in an easily oxidized environment for a long time.
4. The preparation process of the oleogel of the present disclosure is simple, and it can be obtained only by homogenizing and mixing the components without the need for a heating step. An oleogel rich in unsaturated fatty acids that is not layered and has a uniform appearance is prepared. Rapid and continuous production in a large scale can be performed by the process of the present disclosure, and oil composition products with different viscoelasticity can be prepared by simply controlling the process conditions, which can meet the application in different foods, health foods, medicines and cosmetics.
5. The composition of the present disclosure has simple components, and does not require the addition of additional stabilizers which avoids the use of thickeners, emulsifiers and organic agents.

### BRIEF DESCRIPTION OF THE DRAWINGS

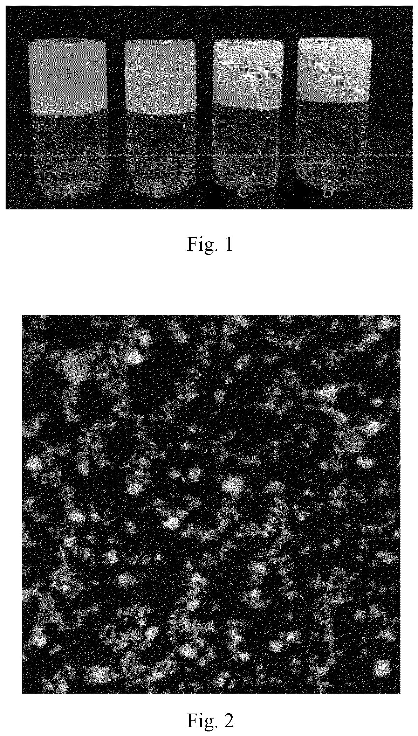

Fig. 1 shows a picture of the appearance of the oleogel. Panel A shows ++++: transparent, not turbid. Panel B shows +++: translucent, substantially not turbid. Panel C shows ++: substantially opaque, slightly turbid. Panel D shows +: opaque, turbid. The more plus signs there are, the more favorable the appearance of the oleogel. The transparency of the oleogel is related to the stability of the product structure. The more transparent it is, the more stable the gel is. The principle is that the higher the degree of dispersion of the protein network in the oleogel, the smaller the size of the protein aggregates, so that the smaller the angle of light scattering in the gel, the more transparent the gel is.
Fig. 2 shows a microscopic picture of the stained oleogel.
Fig. 3 shows the water solubility experiment 1 of the oleogel prepared by different methods.
Fig. 4 shows the water solubility experiment 2 of the oleogel prepared by different methods.

### Detailed Description of the invention

In order to further understand the present disclosure, the preferred embodiments of the present disclosure are described below in conjunction with examples, but it should be understood that these descriptions are only for further illustrating the features and advantages of the present disclosure, rather than limiting the claims of the invention. Those skilled in the art can refer to the content herein and appropriately improve the process parameters to achieve it. It is particularly important to point out that all similar substitutions and modifications are obvious to those skilled in the art, and they are all considered to be included in the present disclosure. The methods and applications of the present disclosure have been described through preferred embodiments, and relevant personnel can obviously modify or appropriately change and combine the methods and applications described herein without departing from the content, spirit and scope of the present disclosure to implement and apply the technology of the present disclosure. Although it is believed that those skilled in the field fully understand the following terms, the following definitions are still stated to help illustrating the subject matter disclosed by the present disclosure.

As used herein, the term "comprising" is synonymous with "including", "containing" or "characterized by", and is inclusive or open-ended, and does not exclude additional undescribed elements or method steps. "Comprising" is a technical term used in the claim language, meaning that the elements are present, but other elements can also be added and still form a structure or method within the scope of the claim.

As used herein, "liquid oil" refers to oil in a fluid state, such as oil in a fluid state at room temperature. Liquid oils may include, but are not limited to, olive oil, canola oil, peanut oil, algae oil, fish oil, linseed oil, perilla seed oil, chia seed oil, and krill oil. Herein, the content of liquid oil is 60-90wt%, such as 65wt%, 70wt%, 75wt%, 80wt%, 85wt%, 89wt%, based on the weight of the oleogel.

As used herein, "unsaturated fatty acids" refer to straight-chain fatty acids containing one or more double bond(s) and a carbon chain which is generally 18-22 carbon atoms in length. Unsaturated fatty acids may include monounsaturated fatty acids and polyunsaturated fatty acids. Polyunsaturated fatty acids are divided into omega-6 series and omega-3 series polyunsaturated fatty acids according to the position and function of the double bond. For example, linoleic acid and arachidonic acid belong to the omega-6 series, and linolenic acid, DHA or EPA belong to the omega-3 series. Preferably, the unsaturated fatty acid is an omega-3 polyunsaturated fatty acid. The content of unsaturated fatty acids contained in the liquid oil may vary. For example, the content of unsaturated fatty acids may be 1%, 2%, 5%, 10%, 15%, 20%, 25%, 30%, 35%, 40%, 45%, 50%, 55%, 60%, 65%, 70%, 75%, 80%, 85%, 90% or 95% or any range or point value there between.

As used herein, "structuring agent" refers herein to a component that aggregate oil through a high molecular weight and network structure caused by strong interactions between molecules. The structuring agent may be a protein denatured particle. The protein may be one or more of an animal protein and a plant protein. For example, the animal protein is one or more of whey protein, casein, collagen and gelatin. Preferably, the structuring agent is a protein denatured particle. The protein denatured particle may be obtained by preparing a protein suspension, heating to denature, homogenizing and drying. In one embodiment, homogenization may be performed by one or more of mechanical stirring homogenization, ultrasonic homogenization, microfluidization homogenization, homogenization with colloid milling, and homogenization with ball milling. In one embodiment, drying can be carried out by one or more of vacuum drying, vacuum freeze drying, electric heating blast drying and spray drying. Preferably, the protein suspension is a whey protein suspension, such as 2-25wt% of whey protein aqueous suspension. For example, the whey protein denatured particle powder can be prepared by preparing a whey protein suspension with a concentration ranging from 2% to 25%, stirring the whey protein suspension with magnetic force to fully dissolve it, and heating it at 75-100°C for 30-100min to prepare a protein denatured particle liquid and drying protein denatured particle liquid. Here, the content of the structuring agent is 6-35wt%, such as 7wt%, 8wt%, 9wt%, 10wt%, 11wt%, 12wt%, 13wt%, 14wt%, 15wt%, 16wt%, 17wt%, 18wt%, 19wt%, 20wt%, 21wt%, 22wt%, 23wt%, 24wt%, 25wt%, 26wt%, 27wt%, 28wt%, 29wt%, 30wt%, 31wt%, 32wt%, 33wt% or 34wt% and any range therebetween, based on the weight of the oleogel.

As used herein, "continuous phase" refers to a substance in which other substances are dispersed. In the oleogel as described herein, the continuous phase can be a liquid oil.

The oleogel of the present disclosure may also contain an appropriate amount of water, such as 4-15wt% of water, such as 5wt%, 6wt%, 7wt%, 8wt%, 9wt%, 10wt%, 11wt%, 12wt%, 13wt% or 14wt% and any range therebetween. In one embodiment, the weight ratio of the structuring agent to water is 1:3-5:1, such as 5:12, 1:2, 7:12, 8:12, 9:12, 10:12, 1:1, 20:12, 25:12, 35:12, or 40:12.

The oleogel of the present disclosure may or may not contain other ingredients other than liquid oil, structuring agent and water. Preferably, the oleogel of the present disclosure does not contain other ingredients. Other ingredients may include, but are not limited to, antioxidants, gels, stabilizers, thickeners or emulsifiers. The antioxidant may include one or more of an oil-soluble antioxidant and a water-soluble antioxidant. The oil-soluble antioxidant may include one or more of rosmarinol, carnosol, sesamol, vitamin E, butylated hydroxyanisole, dibutyl hydroxytoluene, propyl gallate and tert-butylhydroquinone, and the water-soluble antioxidant may include one or more of vitamin C, sodium isoascorbate, tea polyphenols, epigallocatechin gallate, sugars and sugar alcohols. Preferably, the sugar is sucrose. Preferably, the sugar alcohol is one or more of xylitol, sorbitol, erythritol and maltitol.

As used herein, "peroxide value" is an indicator of the degree of oxidation of oil and fatty acids, usually the active oxygen content in 1 kg of sample, expressed in millimoles of peroxide, to indicate whether the sample has deteriorated due to oxidation. Generally speaking, the higher the peroxide value, the higher the degree of rancidity. The peroxide value is the maximum limit set in the food standard for the oxidation of food. If the value is exceeded, the food is unqualified. If the peroxide value exceeds the standard, it means that the food has deteriorated, and deteriorated food may have adverse effects on the human body. The peroxide value can be determined by titration and colorimetry, such as mentioned in the Chinese Food Quality Standard GB/T 5009.37-2003 Method for analysis of hygienic standard of edible oils. The peroxide value standards of different oil are different. For example, for DHA algae oil, the peroxide value/(mmol/kg) should be less than 7.5 (as specified in the Food Industry Standard of the People's Republic of China LS/T 3243-2015); for edible crude vegetable oil, the peroxide value/(g/100g) should be less than 0.25 (as specified in the National Food Safety Standard for Vegetable Oil GB2716-2018); for edible hydrogenated oil, the peroxide value/(g/100g) should be less than 0.1, and for margarine, shortening, cocoa butter substitute, non-dairy cream, powdered oil, etc., the peroxide value/(g/100g) should be less than 0.13 (as specified in the National Food Safety Standard for Edible Oil Products GB15196-2015). For cakes and bread, biscuits, instant noodles, and quick-frozen prepared products, the peroxide value/(g/100g) should be less than 0.25 (as specified in the corresponding national food safety standards). Most of the products in the prior art focus on the oleogel forming properties of the structuring agent, and rarely focus on whether the formed oleogel meets the food standard for the peroxide value. The inventors have found in long-term research that the oleogel of the present disclosure can have a lower peroxide value and meet the national peroxide value requirements for various types of food. For example, the oleogel of the present disclosure can be used as a nutritional supplement in cakes and bread. When appropriate, it can also be used as a nutritional supplement in the above-mentioned and other types of products.

As used herein, viscoelasticity can be characterized by multiple parameters, such as storage modulus value (G'), and/or loss factor (tan δ). Preferably, viscoelasticity can be characterized by loss factor (tan δ) to indicate that the researched sample exhibits solid properties (elasticity) or liquid properties (viscosity). For a completely elastic material, the tan δ value is 0; when 0< tan δ <1, the research sample exhibits greater elasticity than viscosity; when tan δ = 1, the research sample is under critical strain, which can be used to measure ductility; when tan δ >1, the research sample exhibits more viscous behavior than elasticity. Herein, the desired result is 1×10³ Pa≤G'≤1×10⁵Pa, 0.08≤tan δ≤0.5, and an oleogel with greater elasticity than viscosity is obtained. Herein, the loss factor is calculated as loss modulus value (G")/storage modulus value (G'), where the storage modulus value (G') and loss modulus value (G") can be measured by a rheometer.

### Product preparation method

The oleogel of the present disclosure can be prepared by mixing a structuring agent, such as whey protein denatured particles, with liquid oil, homogenizing them to form an oil phase suspension, and then adding water. For whey protein denatured particles, a whey protein suspension with a concentration ranging from 2% to 25% can be prepared, and after being fully dissolved by magnetic stirring, it can be heated at 75 to 100° C for 30 to 100 minutes to prepare a protein denatured particle liquid; after drying, a protein denatured particle powder is obtained. 6 to 35% of whey protein denatured particles can be mixed with liquid oil, such as algae oil rich in Omega-3 polyunsaturated fatty acids, and homogenized to obtain an oil phase suspension of whey protein. Then, 4 to 15% of water can be added to the above suspension, and then homogenized to obtain the oleogel of the present disclosure. The method may not include the step of heating the liquid oil. Herein, heating can be higher than room temperature for a period of time, such as heating at 40°C, 45°C, 50°C, 55°C, 60°C, 65°C, 70°C, 75°C, 80°C, 85°C, 90°C, 95°C, 100°C or higher, such as 30 minutes, 35 minutes, 40 minutes, 45 minutes, 50 minutes, 55 minutes or 60 minutes or longer.

### Product application

The oleogel of the present disclosure can have different effects depending on the type of unsaturated fatty acids contained. For example, the oleogel of the present disclosure can contain one or more of fish oil, squalene, ethyl polyenoate or evening primrose oil, and have the efficacy of these ingredients. The oleogel of the present disclosure can be prepared into an oral pharmaceutical preparation for achieving a variety of therapeutic benefits.

Fish oil is a common animal source of ω-3 and is rich in essential fatty acids DHA and EPA that are beneficial to the human. As a functional oil, fish oil can be used in medicines. Its main functions and effects include: regulating blood lipids, clearing thrombi, preventing blood coagulation, preventing cerebral thrombosis, cerebral hemorrhage and stroke; preventing arthritis, relieving gout and asthma, and temporarily relieving swelling and pain caused by arthritis; preventing senile dementia, nourishing the brain, and improving memory; improving vision, preventing and treating presbyopia; and maintaining the retina. In addition, it can also be used as an auxiliary treatment for diseases. It is suitable for patients with thrombosis, cerebral hemorrhage or stroke; people with three highs (high blood pressure, high blood lipids, and high cholesterol); people with vision loss and presbyopia; people with symptoms of heart disease and arteriosclerosis; people with arthritis, gout, and asthma; and patients who need to prevent and treat complications of diabetes.

Squalene is a natural compound found in shark liver oil. It has multiple physiological functions such as increasing the activity of superoxide dismutase (SOD) in the body, enhancing the body's immune ability, improving sexual function, anti-aging, anti-fatigue, and anti-tumor. Among marine fish oil products, squalene is the only drug that has been confirmed to have the effect of preventing and treating tumors and is widely used in tumor patients. The main effects of squalene are: liver protection, promoting liver cell regeneration and protecting liver cells, thereby improving liver function; anti-fatigue and enhancing the body's disease resistance, improving human immune function; protecting the function of the adrenal cortex and improving stress resistance; anti-tumor, especially after cancer resection surgery or when using radiotherapy and chemotherapy. The effect is remarkable, and its biggest feature is to prevent cancer from metastasizing to the lungs; increase the effect of white blood cells. The application of squalene includes improving the immune system; regulating the acid-base balance of the human body (alkaline products); being used as an antioxidant for effectively improving skin color and relieve skin diseases such as psoriasis and dermatitis. Squalene helps to treat heart disease, high blood pressure, low blood pressure, diabetes, stomach disease, kidney disease, conjunctivitis, empyema, women's diseases, rheumatoid arthritis. Squalene helps people who eat a lot of greasy food to restore the normal level of cholesterol in the blood. Squalene is a fitness tonic.

Ethyl polyenoate is a product of polyunsaturated fatty acid ethyl ester obtained by refining and esterifying fish oil extracted from marine fish. The main ingredients are ethyl eicosapentaenoate and ethyl docosahexaenoate, both of which contain more unsaturated bonds. It is a light yellow to yellow clear oily liquid with a slight fishy smell. It has the effect of lowering serum triglycerides and total cholesterol and is used for hyperlipidemia.

The main ingredients of evening primrose oil are: r-linolenic acid, magnesium, zinc, copper, vitamins C, E, B6, B5. It can be blended with base oils and essential oils, and has multiple therapeutic and whitening functions. It can be made into capsules for oral administration to treat cardiovascular diseases, premenstrual syndrome, and menopausal syndrome. It can be used in aromatherapy to blend lotions and creams, improve eczema, psoriasis, help wound healing and nail development, and solve hair problems. It is generally used at a dose of 10%.

The present disclosure will be further explained in detail with reference to the following examples. However, it should be understood by those skilled in the art that these examples are provided for illustrative purposes only and are not intended to limit the present disclosure.

### Examples

The particular embodiment of present application will be described in detail with reference to the examples as follows. Those skilled in the art will understand that the following examples are only used to illustrate the present application and should not be considered as limiting the scope of the present application. If no specific conditions are specified in the examples, they are carried out under conventional conditions or conditions recommended by the manufacturer. The reagents or instruments used without indicating the manufacturer are all conventional products that can be obtained commercially. Unless otherwise specified, all amounts listed are described in weight percentage based on the total weight. The present application should not be construed as being limited to the specific examples described.

### 1. Preparation method

Step 1: A whey protein suspension with a concentration ranging from 2% to 25% was formulated and fully dissolved by magnetic stirring, heated at 75-100°C for 30-100 minutes to prepare a protein denatured particle liquid. After the protein denatured particle liquid was dried, a protein denatured particle powder was obtained.

Step 2: 6-35% of whey protein denatured particles were mixed with algae oil rich in omega-3 polyunsaturated fatty acids, and homogenized to obtain an oil phase suspension of whey protein;

Step 3: 4-15% of water was added to the above suspension, and then homogenized to obtain an oleogel rich in omega-3 polyunsaturated fatty acids with oil content of up to 60-90%.

### 2. Determination methods

### ① Rheological properties

### Instrument used: Rheometer

Determination steps: The rheological properties of the oleogel were analyzed by small amplitude oscillatory shear analysis. The oleogel was placed on the specimen stage of the rheometer (HAAKE MARS 60), and a sawtooth plate (P35TI/SE) was selected for determination. The gap between the specimen stage and the plate in parallel is 1 mm. First, the sample is strain scanned at 25°C, and the storage modulus (G'), loss modulus (G") and loss factor (tan δ) are recorded to characterize the rheological properties of the oil composition.

For rheological determination, the storage modulus (G') value can evaluate the elasticity measure or storage energy; the loss modulus (G") value can evaluate the viscosity measure or dissipated energy. The loss factor tan δ=G"/G'.

The storage modulus (G') reflects the elasticity of the composition.

Viscoelasticity can be characterized by the loss factor (tan δ) to indicate whether the researched sample exhibits solid properties (elasticity) or liquid properties (viscosity). For a completely elastic material, the tan δ value is 0. When 0<tan δ <1, the elasticity of the researched sample is greater than the viscosity. When tan δ = 1, the researched sample is at a critical strain and can be used to measure ductility. When tan δ >1, the researched sample shows more viscous properties than elasticity.

### ② Oxidation

### Instruments used: acid burette, iodine volumetric flask, rotary evaporator

### Test steps:

### (1) Sampling

The prepared sample were crushed and mixed thoroughly, then placed in a wide-mouth bottle. Petroleum ether with a volume of 2~3 times the volume of the sample was added to the wide-mouth bottle. The wide-mouth bottle was shaken well to mix thoroughly, and stand for more than 12 hours. Filtration was preformed through a funnel filled with anhydrous sodium sulfate to obtain the filtrate. The petroleum ether was evaporated under reduced pressure using a rotary evaporator in a water bath below 40°C. The residue is the sample to be tested.

### (2) Sample determination

Sample determination was performed during which direct sunlight should be avoided. 2g~3g of the sample (accurate to 0.001g) was weighed, placed in a 250mL iodine volumetric flask. 30mL of chloroform-glacial acetic acid mixture was added, and the flask was shaken gently to completely dissolve the sample. 1.00mL saturated potassium iodide solution was accurately added. The bottle cap was plugged tightly. The flask was shaken gently for 0.5min, and placed in the dark for 3min. The flask was taken out and 100mL of water was added. The flask was shaken well. The precipitated iodine was immediately titrated with sodium thiosulfate standard solution (when the peroxide value is estimated to be 5.91mmol/kg or less, 0.002mol/L standard solution was use. When the peroxide value is estimated to be greater than 5.91mmol/kg g, 0.01mo|/L standard solution was used), until that light yellow appeared. 1mL of starch indicator was added. Titration was continued. The flask was shaken vigorously until the blue color of the solution disappeared. A blank test was performed at the same time. The volume V0 of O.01mol/L of sodium thiosulfate solution consumed in the blank test shall not exceed 0.1mL.

### (3) Expression of analytical results

When the peroxide value is expressed as the millimole of active oxygen in 1kg of sample, it is calculated by the formula which is: X = (V -V0) x c/(2 x m) x 1000.

In the formula:
X: peroxide value, in millimoles per kilogram (mmol/kg);
V: volume of sodium thiosulfate standard solution consumed by the sample, in milliliters (mL);
V0: volume of sodium thiosulfate standard solution consumed by the blank test, in milliliters (mL);
c: concentration of sodium thiosulfate standard solution, in moles per liter (mol/L).
m: sample mass, in grams (g);
1000: conversion factor.

The calculation result is expressed as the arithmetic mean of two independent determination results obtained under repeatability conditions, and the result retains two significant figures.

Evaluation criteria: The antioxidant capacity of the oil composition is characterized by the peroxide value. The lower the peroxide value, the better the antioxidant property.

### ③ Appearance evaluation

As shown in Fig. 1, Panel A shows ++++, indicating: transparent, not turbid;
Panel B shows +++, indicating: translucent, substantially not turbid;
Panel C shows ++, indicating: substantially opaque, slightly turbid;
Panel D shows +, indicating: opaque, turbid.

### Example 1: Structural characterization of an oil composition

1. Oleogel was prepared according to the composition in Table 1. Specifically, 18wt% of whey protein denatured particles were added to 70wt% of algae oil rich in omega-3 polyunsaturated fatty acids, mixed for 0.5~3h to obtain an oil phase suspension of protein. 12wt% of purified water was then added to the above oil phase suspension, homogenized for 3~30min to obtain the oleogel rich in omega-3 polyunsaturated fatty acids.

**Table1: Composition of oleogel**

| composition | wt% |
|---|---|
| whey protein denatured particles | 18 |
| algae oil | 70 |
| purified water | 12 |

### The oleogel was observed microscopically (staining method) for structural characterization. The protein particles were fluorescently stained with thioflavin T (Th T) during the preparation process. The image of the protein particles or network was captured using a Leica TCS SP5 confocal laser scanning microscope (Leica Microsystems Inc., Heidelberg, Germany), with an excitation wavelength of 458 nm.

Fig. 2 shows the result of the protein particles forming a permeable network in the oil composition. As shown in Fig. 2, the black part is oil, and the protein has fluorescence absorption after staining, showing green bright spots under the laser confocal microscope. According to the microscopic picture, the protein particles showing green fluorescence are linked and aggregated into chains, and this structure can condense the liquid oil into a semi-solid/solid oil composition, namely the oleogel. The oleogel with this special structure can effectively block oxygen and slow down the oxidative rancidity of oils rich in omega-3 polyunsaturated fatty acids, as shown below. According to Fig. 2, in the oleogel, algae oil is a continuous phase, and protein particles are a dispersed phase.
2. Comparative example of glyceryl mono- and di-stearate structured oil.

90wt% of algae oil and 10wt% of glyceryl mono- and di-stearate were fully mixed, and the mixture was heated at 70-100°C for 30-60min and then cooled down to obtain a oil composition.

Glyceryl mono- and di-stearate was heated and then cooled down to be crystallized in order to obtain a solid/semi-solid oil composition. Since the heating process accelerates the oxidative rancidity of oils rich in omega-3 polyunsaturated fatty acids, the peroxide value is 5.2mmol/kg as determined by oxidation determination. In contrast, the preparation process of the oleogel of the present disclosure does not require a heating step, thus avoiding the oxidation of oil caused by the heating process.

### Example 2: Investigation of different amounts of protein denatured particles added

The content of whey protein denatured particles in the composition of the oleogel was evaluated on the rheological properties and peroxide value of the oleogel. Specifically, the mass of algae oil and water was controlled (70g and 12g, respectively). The mass of the protein denatured particles was changed (in g). The rheological properties and peroxide value were determined by the method described above. The results are shown in Table 2.

**Table 2: Effect of different addition amounts of protein denatured particles on rheological properties and peroxide value**

| composition (in g) | Oleogel No | | | | | | | |
|---|---|---|---|---|---|---|---|---|
| | 1 | 2 | 3 | 4 | 5 | 6 | 7 | 8 |
| algae oil | 70 | 70 | 70 | 70 | 70 | 70 | 70 | 70 |
| protein denatured particles | 3 | 6 | 10 | 20 | 25 | 35 | 40 | 45 |
| Purified water | 12 | 12 | 12 | 12 | 12 | 12 | 12 | 12 |
| G'(*10⁴Pa) | Unformed | 0.6 | 0.8 | 3.7 | 4.2 | 3.6 | 3.4 | Protein precipitation, unable to form a homogeneous system |
| tan δ | | 0.41 | 0.25 | 0.17 | 0.09 | 0.12 | 0.13 | |
| Peroxide Value mmol/kg | | 2.44 | 2.18 | 1.47 | 1.43 | 1.54 | 1.71 | |
| Appearance description | | +++ | ++++ | +++ | +++ | + | + | |

Unformed: refers to the composition after preparation being in liquid or obviously flowing suspension oil, which cannot be coagulated into a solid or semi-solid state.

As shown in Table 2, when the content of protein denatured particles is 6-35%, oleogel can be prepared, and when the content of protein denatured particles is less than 6% or greater than 35%, oleogel cannot be formed. The prepared oleogel has better antioxidant properties, and the peroxide value is less than 2.5mmol/kg, which is suitable for use as food. The inventors found that protein denatured particles have an impact on the appearance of oleogel. When the content of protein denatured particles is 10.8%, the appearance of the oleogel is transparent and not turbid. When the content of protein denatured particles is further increased, the appearance of the oleogel becomes gradually turbid.

Within a certain range of 6-35%, as the content of protein microparticles increases, the gel strength increases. After the content of protein microparticles exceeds a certain range, the protein microparticles are in an oversaturated state and cannot be well dispersed in the oil, resulting in the precipitation of protein microparticles. When the ratio of protein denatured particles to purified water = 3:12 or 45:12, oleogel cannot be formed. Surprisingly, as the ratio of protein denatured particles to purified water increases from 3:12 to 25:12, the peroxide value tends to gradually decrease, and then when it is further increased from 25:12 to 45:12, the peroxide values gradually increase again. When the ratio of protein denatured particles to purified water is 25:12, the peroxide value of the oleogel is the lowest.

### Example 3: Investigation of different algae oil addition amounts

The rheological properties and peroxide value of the oleogel were evaluated by the content of algae oil in the composition of the oleogel. A composition with a lower peroxide value was selected. The ratio of the protein denatured particles to purified water was 20:12 to determine the amount of algae oil added. Specifically, the mass of the protein denatured particles and water was controlled (20g and 12g, respectively). The mass of the algae oil was changed (in g). The rheological properties and peroxide value were determined by the method described above. The results are shown in Table 3.

**Table 3: Effect of different algae oil addition amounts on rheological properties and peroxide value**

| composition (g) | oleogel No | | | | | | | | |
|---|---|---|---|---|---|---|---|---|---|
| | 9 | 10 | 11 | 12 | 13 | 14 | 15 | 16 | 17 |
| algae oil | 30 | 50 | 65 | 80 | 120 | 160 | 200 | 250 | 280 |
| protein denatured particles | 20 | 20 | 20 | 20 | 20 | 20 | 20 | 20 | 20 |
| Purified water | 12 | 12 | 12 | 12 | 12 | 12 | 12 | 12 | 12 |
| G'(*10⁴Pa) | Protein precipitati on, unable to form a homogene ous system | 3.7 | 4.0 | 3.4 | 2.1 | 1.8 | 0.9 | 0.6 | Unformed |
| tan δ | | 0.18 | 0.16 | 0.21 | 0.26 | 0.35 | 0.40 | 0.51 | |
| Peroxide Value mmol/kg | | 1.48 | 1.46 | 1.52 | 1.84 | 2.11 | 2.50 | 3.18 | |
| Appearance description | | + | +++ | +++ | +++ | ++++ | ++++ | +++ | |

As shown in Table 3, when the algae oil content is 60-90%, an oil composition can be prepared, and the prepared oleogel has good antioxidant properties. As the algae oil content increases, the oleogel becomes more transparent. When the ratio of the protein denatured particles to purified water is 20:12, the algae oil content is as high as 89%.

### Example 4: Investigation of different water addition amounts

Evaluation of the effect of water content in the oleogel formula on the rheological properties and peroxide value of the oleogel was performed. Specifically, the mass of protein denatured particles and algae oil was controlled (20g and 70g, respectively). The mass of water was changed (in g), and the rheological properties and peroxide value were determined by the method described above. The results are shown in Table 4.

**Table 4: Effect of different water addition amounts on rheological properties and peroxide value**

| composition(g) | Oleogel No | | | | | |
|---|---|---|---|---|---|---|
| | 18 | 19 | 20 | 21 | 22 | 23 |
| algae oil | 70 | 70 | 70 | 70 | 70 | 70 |
| protein denatured particles | 20 | 20 | 20 | 20 | 20 | 20 |
| Purified water | 3 | 4 | 12 | 15 | 16 | 20 |
| G'(*10⁴Pa) | Unformed | 1.0 | 3.5 | 2.8 | 2.6 | Unformed |
| tan δ | | 0.21 | 0.17 | 0.27 | 0.36 | |
| Peroxide Value mmol/kg | | 2.20 | 1.45 | 1.64 | 1.76 | |
| Appearance description | | +++ | +++ | +++ | ++ | |

As shown in Table 4, when the water content is 4-15%, an oil composition can be prepared, and the prepared oleogel has good antioxidant properties. Surprisingly, the inventors noticed that the water content has an effect on the appearance of the oleogel. When the water content is 4.3%, the oleogel is translucent and substantially not turbid. When the water content increases to 15.1%, the oleogel tends to become more turbid, substantially opaque and turbid.

### Example 5: Investigation of oleogels prepared with different oil

The applicability of the oleogel of the present disclosure to different liquid oils was also investigated. Specifically, the composition of the oleogel is shown in Table 5, and the preparation method was the same as the method described above, except that different liquid oils were added.

**Table 5: Rheological properties and peroxide values of oleogels prepared with different oil**

| composition(g) | Oleogel No | | |
|---|---|---|---|
| | 24 | 25 | 26 |
| linseed oil | 70 | / | / |
| algae oil | / | 70 | / |
| Fish oil | / | / | 70 |
| protein denatured particles | 20 | 20 | 20 |
| Purified water | 8 | 8 | 8 |
| G'(*10⁴Pa) | 3.4 | 3.5 | 3.6 |
| tan δ | 0.17 | 0.17 | 0.16 |
| Peroxide Value mmol/kg | 1.70 | 1.80 | 1.50 |
| Appearance description | +++ | +++ | +++ |

As shown in Table 5, in addition to algae oil, linseed oil and fish oil can also be used to prepare oil compositions in the same way, and the prepared oleogel has good antioxidant properties.

### Example 6: Investigation of adding antioxidants

The effect of adding antioxidants on the rheological properties and peroxide value of the oleogel of the present disclosure was also investigated. Specifically, the composition of the oleogel is shown in Table 6, and the preparation method was the same as the method described above, except that antioxidants, vitamin C (VC), sucrose and erythritol were additionally added. The results are shown in Table 6.

**Table 6: Effect of adding antioxidants on the rheological properties and peroxide value of the oleogel of the present disclosure**

| composition (g) | Oleogel No | | | | | | |
|---|---|---|---|---|---|---|---|
| | control 1 | control 2 | 27 | 28 | 29 | 30 | 31 |
| protein denatured particles | / | / | 20 | 20 | 20 | 20 | 20 |
| algae oil | 70 | 70 | 70 | 70 | 70 | 70 | 70 |
| water | / | / | 8 | 8 | 8 | 8 | 8 |
| monoglyceride | / | 7.8 | / | / | / | / | / |
| VC | / | / | / | 2.5 | 2.5 | 2.5 | 5 |
| sucrose | / | / | / | / | 10 | / | 10 |
| Erythritol | / | / | / | / | / | 10 | / |
| Peroxide Value mmol/kg | 3.52 | 5.26 | 1.80 | 1.50 | 1.24 | 1.21 | 0.83 |
| peroxide value after one month Acceleration* (mmol/kg) | 21.50 | 35.10 | 2.45 | 2.20 | 2.12 | 2.13 | 1.50 |
| Appearance description | / | / | +++ | +++ | +++ | +++ | +++ |

*Sample stability under accelerated test conditions: temperature 40°C, humidity 75% for one month

As shown in Table 6, the oleogel of the present application has better antioxidant properties than control 1 (algae oil only) and control 2 (algae oil and monoglyceride). After adding antioxidants, the antioxidant capacity of the oleogel is improved, but even without adding antioxidants the oleogel of the present disclosure also achieves the same order of magnitude of peroxide value and can be used directly as food.

Example 7: Difference in properties between krill oil oleogel with oil phase as continuous phase and krill oil emulsion gel with water phase as continuous phase

A: Krill oil oleogel was prepared according to the ratio of the composition in Table 1, except that krill oil was used to replace algae oil.

B: Whey protein was used as a gelling agent to prepare krill oil emulsion gel. Specifically, the krill oil emulsion gel was prepared as follows: 1. 300g of 8% whey protein solution was prepared. 2. The aqueous phase was sheared with a high speed at 18000rpm. 200g of krill oil was add. The shearing speed was maintained for 5min to form a uniform krill oil emulsion. 3. The emulsion was heated at 85°C for 30min, and then placed in ice water to be cooled down to form a krill oil emulsion gel.

0.5g of each of the two gels A and B was taken in 50mL of purified water at 60°C and stirred fully for 10min at a speed set to 500rpm. The states of the two gels were observed and compared.

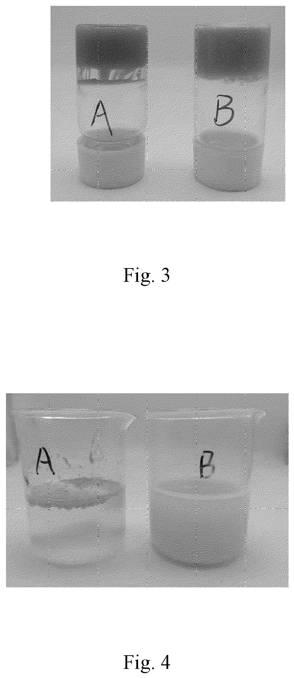

As shown in Figs 3 and 4, although the krill oil oleogel (tube A) and the emulsion gel (tube B) are similar in appearance, the krill oil oleogel is insoluble in water and suspended on water. In contrast, the krill oil emulsion gel can be re-dissolved in water to form an emulsion. From the structural analysis, the continuous phase of the oleogel is oil, while the continuous phase of the emulsion gel is water. When fat-soluble pigments were used for dyeing, the oleogel can be dyed. The emulsion gel is an oil-in-water system, and the fat-soluble pigments cannot penetrate into it, so it cannot be dyed, which is obviously different from the oleogel.

Example 8: Rheological properties and peroxide values of oleogels prepared with different types of proteins

The applicability of different proteins to the oleogel of the present disclosure was also investigated. Specifically, the composition of the oleogel is shown in Table 7, and the preparation method was the same as the method described above, except that different types of proteins were used.

**Table 7: Effects of different types of proteins on the rheological properties and peroxide value of oleogel**

| composition (g) | Oleogel No | | | | | |
|---|---|---|---|---|---|---|
| | 32 | 33 | 34 | 35 | 36 | 37 |
| algal oil | 70 | 70 | 70 | 70 | 70 | 70 |
| whey protein | 20 | / | / | / | / | / |
| casein | / | 20 | / | / | / | / |
| collagen protein | / | / | 20 | / | / | / |
| gelatin | / | / | / | 20 | / | / |
| zein | / | / | / | / | 20 | / |
| mung bean protein | / | / | / | / | / | 20 |
| Purified water | 8 | 8 | 8 | 8 | 8 | 8 |
| G'(*10⁴Pa) | 3.5 | 3.2 | 2.9 | 2.6 | 3.3 | 2.7 |
| tan δ | 0.17 | 0.21 | 0.23 | 0.26 | 0.18 | 0.24 |
| Antioxidant activity mmol/kg | 1.80 | 2.14 | 2.32 | 2.44 | 1.92 | 2.38 |
| Appearance description | +++ | +++ | +++ | +++ | +++ | +++ |

As shown in Table 7, in addition to whey protein, casein, collagen, gelatin, zein and mung bean protein can also be used to prepare oil compositions in the same way, and the prepared oleogel has good antioxidant properties. In the present application, various proteins such as casein, collagen, gelatin, zein and mung bean protein can be used to replace whey protein.

The above embodiments are preferred embodiments of the present disclosure, but the embodiments of the present disclosure are not limited to the above embodiments. Any other changes, modifications, substitutions, combinations, and simplifications that do not deviate from the spirit and principle of the present disclosure should be equivalent replacement methods and are included in the protection scope of the present disclosure.

## Claims

1. An oleogel comprising 60-90 wt% of a liquid oil, 6-35 wt% of a structuring agent and 4-15 wt% of water, wherein the liquid oil comprises unsaturated fatty acids, the structuring agent is a protein denatured particle, and the liquid oil is a continuous phase.

2. The oleogel according to claim 1, wherein the content of the liquid oil is 63-85wt%; and/or the content of the structuring agent is 6-25wt%.

3. The oleogel according to claim 1, wherein the unsaturated fatty acids are one or more of monounsaturated fatty acids and polyunsaturated fatty acids; the monounsaturated fatty acids include one or more of myristic acid, palmitoleic acid, oleic acid, trans-oleic acid, ricinoleic acid, erucic acid and cetoleic acids; the polyunsaturated fatty acids include one or more of omega-3 and omega-6 polyunsaturated fatty acids.

4. The oleogel according to claim 1, wherein the protein is one or more of animal proteins and plant proteins; wherein the animal proteins include one or more of whey protein, casein, collagen and gelatin.

5. The oleogel according to claim 1, wherein the protein denatured particles are obtained by preparing a protein suspension, heating to denature, homogenization and drying; wherein the homogenization is performed by one or more of mechanical stirring homogenization, ultrasonic homogenization, homogenization with microfluidization, homogenization with colloid milling, and homogenization with ball milling; wherein the drying is performed by one or more of vacuum drying, vacuum freeze drying, electric heating blast drying and spray drying.

6. The oleogel according to claim 5, wherein the protein suspension is 2-25wt% of whey protein aqueous suspension.

7. The oleogel according to claim 1, wherein the liquid oil is one or more of olive oil, canola oil, peanut oil, algae oil, fish oil, linseed oil, perilla seed oil, chia seed oil and krill oil.

8. The oleogel according to claim 1, which contains or does not contain an antioxidant; wherein the antioxidant is one or more of an oil-soluble antioxidant and a water-soluble antioxidant.

9. The oleogel according to claim 8, wherein the oil-soluble antioxidant is one or more of rosmarinol, carnosol, sesamol, vitamin E, butylated hydroxyanisole, dibutyl hydroxytoluene, propyl gallate and tert-butylhydroquinone, and the water-soluble antioxidant is one or more of vitamin C, sodium isoascorbate, tea polyphenols, epigallocatechin gallate, sugars and sugar alcohols; wherein the sugars include sucrose; and the sugar alcohols are one or more of xylitol, sorbitol, erythritol and maltitol.

10. The oleogel according to claim 1, wherein the oleogel has one or more of the following characteristics: (1) having a storage modulus value of 1×10³Pa-1×10⁵Pa; and (2) having a loss factor of 0.08-0.5, as measured by a rheometer.

11. Use of the oleogel according to claim 1 in food, health products or cosmetics, or use of the oleogel according to claim 1 in the preparation of oral pharmaceutical preparations, wherein the oral pharmaceutical preparations are used for one or more of the following: (1) regulating blood lipids and/or clearing thrombi; (2) preventing Alzheimer's disease, nourishing the brain and/or improving memory; (3) preventing arthritis, relieving gout and asthma, and temporarily relieving swelling and pain caused by arthritis; and (4) improving vision and/or preventing and treating presbyopia; and (5) maintaining the retina.

12. An oral pharmaceutical preparation comprising the oleogel according to claim 1.

13. A method for preparing the oleogel according to claim 1, comprising: (1) adding a structuring agent to a liquid oil and mixing them to obtain an oil phase suspension of the structuring agent; and (2) adding water to the oil phase suspension of the structuring agent and homogenizing them to obtain an oleogel.

14. The method according to claim 13, further comprising the step of adding an oil-soluble antioxidant, a water-soluble antioxidant, or both to the oil phase, the water phase, or both, respectively; and the method does not include the step of heating the liquid oil.
